# EUROPÄISCHE PATENTSCHRIFT

(11) **EP 0 692 980 B1**
(45) Veröffentlichungstag und Bekanntmachung des Hinweises auf die Patenterteilung: **21.04.1999**
(21) Anmeldenummer: 94912440.8
(22) Anmeldetag: 29.03.1994
(51) Int. Cl.: A61K 51/00

(54) **CHELATBILDNER VOM TYP S3N2 FÜR RADIOAKTIVE ISOTOPE, DEREN METALLKOMPLEXE UND IHRE VERWENDUNG IN DIAGNOSTIK UND THERAPIE**
TYPE S3N2 CHELATORS FOR RADIOACTIVE ISOTOPES, THEIR METAL COMPLEXES AND THEIR DIAGNOSTIC AND THERAPEUTICAL USE
AGENTS CHELATEURS DE TYPE S3N2 D'ISOTOPES RADIOACTIFS, LEURS COMPLEXES METALLIQUES ET LEURS UTILISATIONS EN DIAGNOSTIQUE ET THERAPEUTIQUE

(30) Priorität: 31.03.1993 DE 4311022
(43) Veröffentlichungstag der Anmeldung: 24.01.1996
(73) Patentinhaber: INSTITUT FÜR DIAGNOSTIKFORSCHUNG GmbH AN DER FREIEN UNIVERSITÄT BERLIN, 14050 Berlin (DE)
(72) Erfinder: HILGER, Christoph-Stephan, D-13353 Berlin (DE); DINKELBORG, Ludger, D-13585 Berlin (DE); KRAMP, Wolfgang, D-13503 Berlin (DE); SCHIER, Hans-Martin, D-15344 Strausberg (DE)
(74) Vertreter: Mager, Knut
(86) Internationale Anmeldenummer: DE9400370
(87) Internationale Veröffentlichungsnummer: WO9422492

(56) Entgegenhaltungen:
- EP-A- 0 064 946
- EP-A- 0 089 143
- EP-A- 0 299 795
- WO-A-93/15771
- TETRAHEDRON, Bd.38, Nr.14, 1982, OXFORD GB Seiten 2055 - 2060 T. LODI ET AL. 'CHIRAL AMINOACID CONTAINING ACYCLIC LIGANDS -I. SYNTHESES AND CONFORMATIONS'
- CHEMICAL ABSTRACTS, vol. 118, no. 5, 1. Februar 1993, Columbus, Ohio, US; abstract no. 33497,
- TETRAHEDRON, Bd.38, Nr.14, 1982, OXFORD GB Seiten 2061 - 2067 R. MARCHELLI ET AL. 'CHIRAL AMINOACID CONTAING LIGANDS-II. COMPLEXATION OF ALKALINE EARTH CATIONS'

## Beschreibung

Die Erfindung betrifft neue bifunktionelle chalkogenatom-unterbrochene Chelatbildner, diese Verbindungen enthaltende pharmazeutische Mittel, ihre Verwendung in der Radiodiagnostik und Radiotherapie sowie Verfahren zur Herstellung dieser Verbindungen und Mittel. Die Anwendungen von Komplexbildnern für radioaktive Isotope bzw. ihre Komplexe mit radioaktiven Metallen in der Radiodiagnostik und Radiotherapie ist seit langem bekannt. Für die Radiodiagnostik wird am häufigsten das Radionuklid Technetium-99m verwendet, das auf Grund seiner günstigen physikalischen Eigenschaften (keine Korpuskularstrahlung, geringe Halbwertzeit von 6.02 h, gute Detektierbarkeit durch 140 KeV γ-Strahlung) und geringen biologischen Halbwertzeit und einfacher Verfügbarkeit besonders gut für eine in vivo Anwendung geeignet ist. Zur Bildung von Technetium-99m-Komplexen wird Pertechnetat zunächst aus einem Nuklidgenerator gewonnen und durch Verwendung geeigneter Reduktionsmittel (z. B. SnCl₂, S₂O₄²⁻ etc.) in eine niedrigere Oxidationsstufe überführt, die anschließend durch einen geeigneten Chelator stabilisiert wird. Da Technetium in einer Reihe von Oxidationsstufen (+7 bis -1) vorliegen kann, die die pharmakologischen Eigenschaften durch Veränderungen der Ladung eines Komplexes stark verändern können, ist es notwendig, Chelatoren bzw. Komplexliganden für Technetium-99m bereitzustellen, die Technetium sicher, fest und stabil in einer definierten Oxidationsstufe binden können, um zu verhindern, daß durch in vivo ablaufende Redoxprozesse bzw. Technetiumfreisetzungen aus dem entsprechenden Radiodiagnostika eine unerwünschte Biodistribution stattfindet, die eine sichere Diagnostik entsprechender Erkrankungen erschwert.

Als geeignete Komplexbildner für Technetium und Rheniumisotope gelten z. B. cyklische Amine (Troutner, D. E. et al.: J. Nucl. Med. 21, 443 (1980)), die aber den Nachteil haben, daß sie erst ab einem pH > 9 in der Lage sind, Technetium-99m in guten Ausbeuten zu binden. N₂O₂-Systeme (Pillai, M. R. A., Troutner, D. E. et al.; Inorg. Chem. 29, 1850 (1990)) befinden sich in der klinischen Anwendung. Nichtcyclische N₄-Systeme, wie z. B. das HMPAO haben als großen Nachteil ihre geringe Komplexstabilität. Tc-99m-HMPAO muß wegen seiner Instabilität (Ballinger, J. R. et al., Appl. Radiat. Isot. 42, 315 (1991)); Billinghurst M. W. et al., Appl. Radiat. Isot. 42, 607 (1991)) sofort nach seiner Markierung appliziert werden, damit der Anteil an Zerfallprodukten, die eine andere Pharmakokinetik und Ausscheidung besitzen, klein gehalten werden kann. Solche radiochemischen Verunreinigungen erschweren die Erkennung von zu diagnostizierenden Erkrankungen. Eine Kopplung dieser Chelate bzw. Chelatbildner an andere, sich selektiv in Krankheitsherden anreichernde Substanzen ist nicht mit einfachen Mitteln zu lösen, so daß sich diese im allgemeinen unspezifisch im Organismus verteilen.

N₂S₂-Chelatoren (Bormans, G. et al.; Nucl. Med. Biol. 17, 499 (1990)) wie z. B. Ethylendicystein (EC; Verbruggen, A. M. et. al.; J. Nucl. Med. 33, 551 (1992)) erfüllen zwar die Forderung nach hinreichender Stabilität des entsprechenden Technetium-99m-Komplexes, bilden aber erst ab einem pH-Wert des Komplexierungsmediums > 9 Radiodiagnostika mit einer Reinheit von größer 69 %. N₃S-Systeme (Fritzburg, A.; EPA 0 173 424 und EPA 0 250 013) bilden zwar stabile Technetium-99m-Komplexe, müssen aber zum Einbau des Radioisotops auf Temperaturen von ca. 100 °C erhitzt werden.

Ein weiterer Nachteil der N₂S₂- und N₃S-Systeme besteht darin, daß diese teilweise rasch und ohne spezifische Anreicherung vom Organismus ausgeschieden werden, so daß diese nur als Nierenfunktionsdiagnostika in der Klinik Anwendung finden und somit eine beschränkte Verwendbarkeit besitzen. In den letzten Jahren ist das Verlangen nach sich spezifisch in erkrankten Geweben anreichernden Radiodiagnostika gestiegen. Dies kann erreicht werden, wenn Komplexbildner leicht an sich selektiv anreichernde Substanzen gekoppelt werden können und dabei ihre günstigen Komplexeigenschaften nicht verlieren. Da es aber sehr häufig dazu kommt, daß nach Kopplung eines Komplexbildners unter Nutzung einer seiner funktionellen Gruppen an ein solches Molekül eine Abschwächung der Komplexstabilität beobachtet wird, erscheinen die bisherigen Ansätze zur Kupplung von Chelatbildnern an sich selektiv anreichernde Substanzen wenig zufriedenstellend, da ein diagnostisch nicht tolerierbarer Anteil des Isotops aus dem Konjugat in vivo freigesetzt wird (Brechbiel, M. W. et al.; Inorg. Chem. 1986, 25, 2772). Es ist deswegen notwendig, bifunktionelle Komplexbildner darzustellen, die sowohl funktionelle Gruppen zur Bindung des gewünschten Metallions als auch eine (andere, mehrere) funktionelle Gruppe zur Bindung des sich selektiv anreichernden Moleküls tragen. Solche bifunktionellen Liganden ermöglichen eine spezifische, chemisch definierte Bindung von Technetium- oder Rhenium-Isotopen an verschiedenste biologische Materialien, auch dann, wenn ein sogenanntes Prelabeling durchgeführt wird. Es wurden einige Chelatbildner, gekoppelt an monoklonale Antikörper (z. B. EP Appl. 0 247 866 und 0 188 256) oder Fettsäuren (EP Appl. 0 200 492), beschrieben. Als Chelatbildner werden jedoch die bereits erwähnten N₂S₂-Systeme verwendet, die auf Grund ihrer geringen Stabilität wenig geeignet sind. Da sowohl die sich selektiv anreichernden Substanzen in ihren Eigenschaften sowie auch die Mechanismen, nach denen sie angereichert werden, sehr unterschiedlich sind, ist es weiterhin notwendig, den kopplungsfähigen Chelatbildner zu variieren und den physiologischen Anforderungen des Kopplungspartners hinsichtlich seiner Lipo- und Hydrophilie, Membranpermeabilität bzw. -impermeabilität etc. anpassen zu können.

Der Erfindung lag daher die Aufgabe zugrunde, stabile Komplexverbindungen, die gekoppelt oder fähig zur Kopplung an unterschiedliche sich selektiv anreichernde Verbindungen sind, zur Verfügung zu stellen, desweiteren solche koppelbaren Chelatoren oder Komplexe bereitzustellen, die über eine größere chemische Variationsbreite der Substituenten verfügen, um diese den oben referierten Erfordernissen anpassen zu können. Weiterhin liegt der Erfindung die Aufgabe zugrunde, derartige Verbindungen und sie enthaltende pharmazeutische Mittel zur Verfügung zu stellen, sowie Verfahren zu ihrer Herstellung zu schaffen.

Überraschenderweise wird diese Aufgabe erfindungsgemäß dadurch gelöst, daß sich die neuen ungewöhnlichen, bifunktionellen, Chalkogenatom -unterbrochenen Chelatbildner und deren Kopplungsprodukte mit sich spezifisch anreichernden Verbindungen hervorragend zur Herstellung von Radiodiagnostika bzw. Radiotherapeutika eignen.

Gegenstand der Erfindung sind Verbindungen der allgemeinen Formel I

M-L (I)

B-CO-CR¹R²-A- CR³R⁴-CO-D (I)

worin A für ein Chalkogenatom O, S oder Se steht;
R¹,R²,R³ und R⁴ gleich oder unterschiedlich sind und jeweils für ein Wasserstoffatom und/oder für einen verzweigten oder unverzweigten C₁-C₆-Alkylrest stehen, B und D gleich oder verschieden sind und einen Rest

   -NH-CR⁵R⁶-(CR⁷R⁸)_{n=1,2}-S-R⁹ darstellen,

   worin
R⁵ und R⁶ gleich oder unterschiedlich sind und jeweils ein Wasserstoffatom oder einen unverzweigten, verzweigten, cyclischen oder polycyclischen C₁-C₆₀-Alkyl-, Alkenyl-, Polyalkenyl-, Alkinyl-, Polyalkinyl-, Aryl-, Alkylaryl-, Arylalkylrest darstellen, welcher gegebenenfalls mit Hydroxy-, Carboxy-, Aminocarbonyl-, Alkoxycarbonyl-, Amino-, Aldehyd-, Oxo-, Oxy- oder Alkoxy-Gruppen mit bis zu 20 Kohlenstoffatomen substituiert ist und/oder gegebenenfalls durch ein oder mehrere Heteroatome aus der Reihe O, N, S, P, As, Se unterbrochen und/oder substituiert ist,
R⁷und R⁸ gleich oder unterschiedlich sind und jeweils für ein Wasserstoffatom und/oder für einen verzweigten oder unverzweigten C₁-C₆-Alkylrest stehen,
R⁹ für ein Wasserstoffatom, für einen verzweigten oder unverzweigten C₁-C₆-Alkylrest oder für eine Schwefelschutzgruppe steht
und R⁹ und R⁵ gegebenenfalls zusammen mit den sie verbindenden Gruppen einen gegebenenfalls mit Hydroxy-, Oxo-, Oxy- oder Alkoxy-Gruppen mit bis zu 6 Kohlenstoffatomen substituierten 4- bis 8-gliedrigen Ring bilden, sowie deren Komplexe mit Radioisotopen von T_{C} oder Re.
Bevorzugte Verbindungen der allgemeinen Formel (I) zeichnen sich dadurch aus, daß R¹, R², R³, R⁴ und R⁵ Wasserstoffatome darstellen.

Besonders bevorzugte Verbindungen der allgemeinen Formel (I) zeichnen sich dadurch aus, daß R¹,R², R³, R⁴, R⁵, R⁷ und R⁸ Wasserstoffatome sind.

Besonders bevorzugt sind erfindungsgemäße Liganden, bei denen R¹, R², R³, R⁴, R⁵, R⁶, R⁷ und R⁸ jeweils für ein Wasserstoffatom stehen.

Ein weiterer Gegenstand der Erfindung sind Konjugate, enthaltend Verbindungen der allgemeinen Formel (I) und sich selektiv in erkrankten Geweben anreichernde Substanzen, wobei zwischen diesen eine kovalente Bindung besteht und diese im Falle von Carboxy- oder Aminogruppen enthaltenden Substanzen wie Peptiden, Proteinen, Antikörpern oder deren Fragmenten, amidisch oder im Falle von Hydroxylgruppen enthaltenden Substanzen wie Fettalkoholen esterartig oder im Falle von Aldehydgruppen enthaltenden Substanzen imidisch vorliegt.

Besonders bevorzugte Konjugate zeichnen sich dadurch aus, daß die sich im erkrankten Gewebe anreichernden Substanzen Peptide wie Endotheline, Teilsequenzen von Endothelinen, Endothelin-Analoga, Endothelin-Derivate oder Endothelin-Antagonisten bedeuten.

In weiteren bevorzugten erfindungsgemäßen Konjugaten weisen die Peptide die folgenden Sequenzen oder Teile davon 
Cys-Ser-Cys-Ser-Ser-Leu-Met-Asp-Lys-Glu-Cys-Val-Tyr-
Phe-Cys-His-Leu-Asp-Ile-Ile-Trp,

Cys-Ser-Cys-Ser-Ser-Trp-Leu-Asp-Lys-Glu-Cys-Val-Tyr-
Phe-Cys-His-Leu-Asp-Ile-Ile-Trp,

Cys-Thr-Cys-Phe-Thr-Tyr-Lys-Asp-Lys-Glu-Cys-Val-Tyr-
Tyr-Cys-His-Leu-Asp-Ile-Ile-Trp,

Cys-Ser-Ala-Ser-Ser-Leu-Met-Asp-Lys-Glu-Ala-Val-Tyr-
Phe-Cys-His-Leu-Asp-Ile-Ile-Trp,

Cys-Ser-Cys-Asn-Ser-Trp-Leu-Asp-Lys-Glu-Cys-Val-Tyr-
Phe-Cys-His-Leu-Asp-Ile-Ile-Trp,
 
Cys-Ser-Cys-Lys-Asp-Met-Thr-Asp-Lys-Glu-Cys-Leu-Asn-
Phe-Cys-His-Gln-Asp-Val-Ile-Trp,

Ala-Ser-Cys-Ser-Ser-Leu-Met-Asp-Lys-Glu-Cys-Val-Tyr-
Phe-Ala-His-Leu-Asp-Ile-Ile-Trp,

Ala-Ser-Ala-Ser-Ser-Leu-Met-Asp-Lys-Glu-Ala-Val-Tyr-
Phe-Ala-His-Leu-Asp-Ile-Ile-Trp,

Cys-Ser-Cys-Ser-Ser-Trp-Leu-Asp-Lys-Glu-Ala-Val-Tyr-
Phe-Ala-His-Leu-Asp-Ile-Ile-Trp,

Cys-Val-Tyr-Phe-Cys-His-Leu-Asp-Ile-Ile-Trp,

N-Acetyl-Leu-Met-Asp-Lys-Glu-Ala-Val-Tyr-Phe-Ala-His-
Leu-Asp-Ile-Ile-Trp,
 die Teilsequenz
His-Leu-Asp-Ile-Ile-Trp
   oder die cyclischen Aminosäuresequenzen
Cyclo-(DTrp-DAsp-Pro-DVal-Leu),
Cyclo-(DGlu-Ala-alloDIle-Leu-DTrp)
auf.

Die Herstellung der erfindungsgemäßen Verbindungen erfolgt dadurch, daß man Technetium-99m oder Re in Form von Pertechnetat oder Perrhenat in Gegenwart eines Reduktionsmittels und gegebenenfalls eines Hilfsliganden mit Verbindung der allgemeinen Formel (I)

B-CO-CR¹R²-A-CR³R⁴-CO-D (I)

worin
R¹, R², R³, R⁴, A, B und D die vorstehend angegebene Bedeutung haben, umsetzt.

Die Herstellung der erfindungsgemäßen Liganden der allgemeinen Formel (I) erfolgt dadurch, daß man Verbindungen der allgemeinen Formel (II)

X-CO-CR¹R²-A-CR³R⁴-CO-X' (II

worin R¹, R², R³, R⁴ und A die voranstehend angegebene Bedeutung haben und X, X' für eine Abgangsgruppe stehen, mit Verbindungen der allgemeinen Formel (III)

   H-B (III)

   und/oder der allgemeinen Formel (IV)

   H-D (IV)

   umsetzt,
wobei B und D die voranstehend angegebene Bedeutung haben.

Diese Umsetzungen werden in polaren und unpolaren, aprotischen Lösungsmitteln wie beispielsweise Dichlormethan, Tetrahydrofuran, Chloroform, 1,4-Dioxan, DMF oder DMSO bei Temperaturen zwischen -30 und +100 °C unter Zugabe einer Hilfsbase zum Abfangen der freiwerdenden Säuren durchgeführt. Solche können beispielsweise sein: tertiäre Amine, Alkali- und Erdalkalihydroxide, Alkali- und Erdalkalicarbonate.

Ein weiterer Gegenstand der Erfindung sind Kits, die zur Herstellung von Radiopharmaka dienen, bestehend aus einer Verbindung der allgemeinen Formel (I), oder einem erfindungsgemäßen Konjugat enthaltend Verbindungen der allgemeinen Formel (I) und sich selektiv in Geweben anreichernden Substanzen, einem Reduktionsmittel und gegebenenfalls einem Hilfsliganden, die in trockenem Zustand oder in Lösung vorliegen, einer Gebrauchsanweisung mit einer Reaktionsvorschrift zur Umsetzung der beschriebenen Verbindungen mit Technetium-99m oder Re in Form einer Pertechnetatlösung oder Perrhenatlösung.

Gegenstand der Erfindung ist auch eine radiopharmazeutische Zusammensetzung zur nicht invasiven in vivo Darstellung von Rezeptoren und rezeptorhaltigem Gewebe und/oder von atherosklerotischen Plaques, die eine Verbindung der allgemeinen Formel (I) oder ein erfindungsgemäßes Konjugat enthaltend Verbindungen der allgemeinen Formel (I ) und sich selektiv in Geweben anreichernden Substanzen, gegebenenfalls mit den in der Galenik üblichen Zusätzen, enthält, wobei die Verbindung in einem Kit mit Technetium-99m oder Re in Form einer Pertechnetatlösung oder Perrhenatlösung zubereitet wird.

In einer Methode zur Durchführung einer radiodiagnostischen Untersuchung wird die radiopharmazeutische Zusammensetzung in einer Menge von 0,1 bis 30 mCi, bevorzugt von 0,5 bis 10 mCi pro 70 Kg Körpergewicht einem Patienten verabreicht und die vom Patienten abgegebene Strahlung aufgezeichnet.

Überraschenderweise zeigen viele der synthetisierten und mit Tc-99m oder Re markierten Chelate eine höhere Stabilität als vergleichbare N₂S₂- und N₃S-Systeme, die in der Literatur beschrieben sind. So konnten z. B. bei einer erfindungsgemäßen Substanz (Beispiel 3 a), die an einen Fettalkohol gekoppelt wurde, keine Zersetzungsprodukte nach 24 h beobachtet werden. Auch konnte durch Kompetitionsversuche festgestellt werden, daß die in dieser Erfindung beschriebene Tc-99m oder Re-Chelatoren besser als die vergleichbaren N₂S₂, N₃S und Propylenaminoxium-Systeme komplexieren. Die in der vorliegenden Erfindung beschriebenen Chelate und Chelatbildner sind damit eindeutig besser für diagnostische und therapeutische Zwecke geeignet als die bisher bekannten Systeme. Ein besonderer Vorteil der erfindungsgemäßen Chelatoren besteht darin, daß deren Synthesen ohne Verwendung von Schwefelschutzgruppen geführt werden können. Dies macht deren Synthese sehr einfach und zusätzlich bieten speziell solche erfindungsgemäß beschriebenen Verbindungen den Vorteil, daß nach radiochemischer Markierung keine weiteren Fremdmoleküle in den zur Radiodiagnostik bzw. Radiotherapie, z. B. intravenös zu applizierenden Lösungen, enthalten sind, die häufig die Biodistribution des Radiopharmakons stören und damit den diagnostischen Informationsgehalt nachteilig beeinflußen können. Außerdem können die Markierungen an solchen Liganden bzw. deren Kopplungsprodukte an sich selektiv in erkrankten Geweben anreichenden Substanzen unter sehr milden Bedingungen vorgenommen werden. So gelingt die Markierung der erfindungsgemäßen Liganden bzw. der Kopplungsprodukte an sich selektiv in erkrankten Geweben anreichernden Substanzen bei Raumtemperatur und bei physologischem pH-Wert, ohne daß vorher unter Einwirkung von Basen, Säuren oder anderen dem Fachmann bekannten Hilfsstoffen, die Schutzgruppen abzuspalten wären. Dies bietet die Gewähr, daß durch solche Hilfsstoffe die häufig sehr empfindlichen, sich selektiv in erkrankten Geweben anreichernden Substanzen nicht chemisch verändert werden, was häufig deren selektive Anreicherung in erkranktem Gewebe herabsetzt und somit den Informationsgehalt bei der Radiodiagnostik nachteilig beeinflussen würde.

Dennoch können natürlich auch hier Schwefelschutzgruppen Verwendung finden, wenn die eben geschilderten Nachteile in Kauf genommen werden können. Deren Etablierung an Schwefelatomen bzw. deren Abspaltung geschieht dann nach Methoden, die dem Fachmann bekannt sind. Die Kopplung an sich selektiv in erkrankten Geweben anreichernden Substanzen erfolgt ebenfalls nach an sich dem Fachmann bekannten Methoden (z. B. Fritzberg et al.; J. Nucl. Med. 26, 7 (1987)), beispielsweise durch Reaktion von elektrophilen Gruppen des Komplexliganden mit nukleophilen Zentren der sich selektiv in erkranten Geweben anreichernden Substanzen. Ansonsten werden nukleophile Gruppen des Chelators mit elektrophilen Gruppen der sich selektiv in erkrankten Geweben anreichernden Substanzen gekoppelt.

Als Kopplungspartner sind u. a. verschiedene Biomoleküle vorgesehen. Liganden, die an spezifische Rezeptoren binden und so ein in ihrer Rezeptordichte verändertes Gewebe erkennen können, hierzu gehören u. a. Peptide und Steroidhormone, Wachstumsfaktoren und Neurotransmitter. Mit Liganden für Steroidhormonrezeptoren wurde die Möglichkeit einer verbesserten Diagnostik von Brust und Prostatacarcinomen aufgezeigt (S. J. Brandes and J. A. Katzenellenbogen, Nucl. Med. Biol. 15, 53, 1988). Verschiedentlich weisen Tumorzellen eine veränderte Dichte von Rezeptoren für Peptidhormone oder Wachstumsfaktoren, wie z. B. den "epidermal growth factor" (EgF) auf. Die Konzentrationsunterschiede konnten zur selektiven Anreicherung von Cytostatika in Tumorzellen genutzt werden (E. Aboud-Pirak et al., Proc. Natl. Acad. Sci. USA 86: 3778, 1989). Vielfach konnten mit Positronen-emittierenden Isotopen markierte Liganden für Neurorezeptoren zur Diagnostik verschiedener Hirnerkrankungen herangezogen werden (J. J. Forst, Trends in Pharmacol. Sci. 7: 490, 1989). Weitere Biomoleküle sind in den Metabolismus der Zellen einschleusbare Metabolite, die einen veränderten Stoffwechsel erkennbar machen; hierzu gehören beispielsweise Fettsäuren, Saccharide, Peptide und Aminosäuren. Fettsäuren gekoppelt an die instabileren N₂S₂-Chelatbildner wurden in der EPA 0 200 492 beschrieben. Andere Stoffwechselprodukte wie Saccharide, (Desoxyglukose), Lactat, Pyruvat und Aminosäuren (Leucin, Methylmethionin, Glycin) wurden mit Hilfe der PET-Technik zur bildlichen Darstellung von veränderten Stoffwechselvorgängen herangezogen (R. Weinreich, Swiss Med. 8, 10, 1986). Auch nicht biologische Substanzen wie Misonidazol und seine Derivate, die sich in Geweben bzw. Gewebeteilen mit reduzierter Sauerstoffkonzentration irreversibel an Zellbestandteile binden, können zur spezifischen Anreicherung von radioaktiven Isotopen und somit zur bildlichen Darstellung von Tumoren oder ischämischen Regionen herangezogen werden. (M. E. Shelton, J. Nucl. Med. 30; 351, 1989). Schließlich ist auch die Kopplung der bifunktionellen Chelatbildner an monoklonale Antikörper bzw. deren Fragmente möglich. Besonders günstig erwiesen sich Kopplungsprodukte der erfindungsgemäßen Chelatoren bzw. deren Komplexe mit Technetium-99m oder Re mit Fettalkoholen, Fettalkoholderivaten oder mit Fettaminen bzw. deren Derivate oder mit Endothelinen, Teilsequenzen von Endothelinen, Endothelin-Analoga, Endothelin-Derivaten oder Endothelin-Antagonisten zur Detektion von atherosklerotischen Gefäßerkrankungen. Die Derivate wurden WHHL-Kaninchen appliziert, die durch einen genetischen Defekt des LDL-Rezeptors hohe LDL-Konzentrationen im Blut aufweisen und somit atherosklerotische Läsionen aufweisen. Etwa 4 bis 5 h nach i. V. Applikation der Derivate in WHHL-Kaninchen konnten Anreicherungsquotienten im Vergleich zu nicht geschädigtem Gewebe von 3 bis 40 in den atheromatösen Plaques nachgewiesen werden. Dadurch können atherosklerotische Gefäßbereiche mit den in der Radiodiagnostik üblichen Methoden (z. B. Gamma-Szintillationskamera) nachgewiesen werden. Bisher konnten nur sehr späte Stadien der Atherogenese mit invasiveren Verfahren (z. B. Arteriographie) diagnostiziert werden. Die erfindungsgemäßen Substanzen bieten deshalb den entscheidenen Vorteil, viel frühere Stadien der Atherosklerose mit nicht invasiven Verfahren zu diagnostizieren.

Es ist unerheblich, ob eine Markierung der Chelatbildner mit Tc-99m oder Rhenium-Isotopen vor oder nach der Kopplung an das sich selektiv anreichernde Molekül durchgeführt wird. Für eine Kopplung an das sich selektiv anreichernde Molekül nach einer Komplexierung ist jedoch Voraussetzung, daß die Umsetzung des radioaktiven Komplexes mit der sich anreichernden Verbindung schnell, unter schonenden Bedingungen und nahezu quantitativ abläuft, so daß keine anschließende Aufreinigung erforderlich ist.

Die Herstellung der erfindungsgemäßen pharmazeutischen Mittel erfolgt in an sich bekannter Weise, in dem man die erfindungsgemäßen Komplexbildner unter Zusatz eines Reduktionsmittels, vorzugsweise Zinn-(II)-salzen wie -chlorid oder -tartrat - und gegebenenfalls unter Zugabe der in der Galenik üblichen Zusätze - in wäßrigem Medium löst und anschließend sterilfiltriert. Geeignete Zusätze sind beispielsweise physiologisch unbedenkliche Puffer (z. B. Tromethamin), geringe Zusätze von Elektrolyten (z. B. Natriumchlorid), Stabilisatoren (z. B. Gluconat, Phosphate oder Phosphonate). Das erfindungsgemäße pharmazeutische Mittel liegt in Form einer Lösung oder in lyophilisierter Form vor und wird kurz vor der Applikation mit einer Lösung Tc-99m-Pertechnetat, eluiert aus kommerziell erhältlichen Generatoren, oder einer Perrhenatlösung versetzt.

Bei der nuklearmedizinischen in-vivo-Anwendung werden die erfindungsgemäßen Mittel in Mengen von 1.10⁻⁵ bis 5.10⁴ nmol/kg Körpergewicht, vorzugsweise in Mengen zwischen 1.10⁻³ bis 5.10² nmol/kg Körpergewicht dosiert. Ausgehend von einem mittleren Körpergewicht von 70 kg beträgt die Radioaktivitätsmenge für diagnostische Anwendungen zwischen 0,05 und 50 mCi, vorzugsweise 5 bis 30 mCi pro Applikation. Für therapeutische Anwendungen werden zwischen 5 und 500 mCi, vorzugsweise 10 bis 350 mCi appliziert. Die Applikation erfolgt normalerweise durch intravenöse, intraarterielle, peritoneale oder intertumorale Injektion von 0,1 bis 2 ml einer Lösung der erfindungsgemäßen Mittel. Bevorzugt ist die intravenöse Applikation.

Die nachfolgenden Beispiele dienen der näheren Erläuterung des Erfindungsgegenstandes.

### Beispiel 1 a

### N,N'-Bis-(2-Mercapto-1-(methoxycarbonyl)-ethyl)-thiodiglycolsäurediamid

Zu einer Lösung aus 17,16 g (0,1 mol) Cysteinmethylesterhydrochlorid und 20,24 g (0,2 mol) Triethylamin in einem Liter wasserfreiem Dichlormethan wird bei 0 °C und unter Argonatmosphäre eine Lösung von 9,35 g (0,05 mol) Thiodiglycolsäuredichlorid, gelöst in 250 ml wasserfreiem Dichlormethan, getropft. Anschließend wird 1 h bei 0 °C und abschließend 16 h bei Raumtemperatur nachgerührt. Zur Aufarbeitung wäscht man die Dichlormethanlösung jeweils dreimal mit 2 %iger Citronensäure, gesättigter Natriumhydrogencarbonatlösung und mit Wasser. Nach Trocknung über Natriumsulfat wird das Produkt durch Verdampfen des Lösungsmittels unter vermindertem Druck gewonnen. Zur Reinigung wird aus Diethylether kristallisiert.

Ausbeute: 15,32 g (79,7 %), weißes Pulver

| Analyse: | | | | | |
|---|---|---|---|---|---|
| Ber.: | C 37,49 | H 5,24 | N 7,29 | O 24,97 | S 25,02 |
| Gef.: | C 37,28 | H 5,41 | N 7,17 | | S 24,89 |

### Beispiel 1 b

### N,N'-Bis-(2-Mercapto-1-(methoxycarbonyl)-ethyl)-thiodiglycolsäurediamid-Technetium-99m-Komplex

10 mg des unter Beispiel 1 a hergestellten Liganden werden in 1,0 ml 25 % EtOH gelöst. 50 µl dieser Ligand-Lösung werden mit 250 µl Phosphat-Puffer pH 9,5, 50 µl einer desoxygenierten wäßrigen Citratlösung (50 mg/ml), 2,5 µl einer desoxygenierten wässrigen Zinn(II)chlorid-Lösung (5 mg/ml 0,05 N HCl) und 100 µl einer Pertechnetat-Lösung (400 - 900 µCi) versetzt. Das Reaktionsgemisch wird nach einer Inkubationszeit von 10 min mittels HPLC auf die Reinheit des gebildeten Tc-Komplexes untersucht: Hamilton PRP-1 Säule, 5 µm, 125 x 4,6 mm; Gradientenelution von 100 % A nach 100 % B innerhalb von 7,5 min (Eluent A: Natriumhydrogenphosphat 0,005 M, pH 7,4; Eluent B: Acetonitril/Natriumhydrogenphosphat 0,005 M, pH 7,4 (75/25); 2,0 ml/min. Die radiochemische Reinheit ist > 98 %.

### Beispiel 2 a

### N,N'-Bis-(2-Mercapto-1-(hydroxycarbonyl)-ethyl)-thiodiglycolsäurediamid

3,84 g (10 mmol) des unter Beispiel 1 a hergestellten Liganden werden in 200 ml 2N Natriumhydroxidlösung unter Argonatmosphäre gelöst. Man rührt 1,5 h bei Raumtemperatur, stellt mit argongesättigter, konzentrierter Salzsäure pH = 2 ein und extrahiert das ausgeschiedene Öl mit argongesättigtem Essigsäureethylester. Nach Trocknung über Natriumsulfat wird das Lösungmittel unter vermindertem Druck verdampft, und das ölige Rohprodukt durch verreiben mit Diethylether kristallisiert.

Ausbeute: 932 mg (26,15 %), weißes Pulver

| Analyse: | | | | | |
|---|---|---|---|---|---|
| Ber.: | C 33,70 | H 4,52 | N 7,86 | O 26,93 | S 26,98 |
| Gef.: | C 33,49 | H 4,73 | N 7,71 | | S 26,73 |

### Beispiel 2 b

### N,N'-Bis-(2-Mercapto-1-(hydroxycarbonyl)-ethyl)-thiodiglycolsäurediamid Technetium-99m-Komplex

10 mg des unter Beispiel 2 a hergestellten Liganden werden in 1,0 ml 0,5 M Phosphat-Puffer pH 7,5 gelöst. 50 µl dieser Ligand-Lösung werden mit 250 µl Phosphat-Puffer pH 7,5, 50 µl einer desoxygenierten wäßrigen Citratlösung (50 mg/ml), 2,5 µl einer desoxygenierten wäßrigen Zinn(II)chlorid-Lösung (5 mg/ml 0,05 N HCl) und 100 µl einer Pertechnetat-Lösung (400 - 900 µCi) versetzt. Das Reaktionsgemisch wird nach einer Inkubationszeit von 10 min mittels HPLC auf die Reinheit des gebildeten Tc-Komplexes untersucht: Hamilton PRP-1 Säule, 5 µm, 125 x 4,6 mm; Gradienteneluation von 100 % A nach 100 % B innerhalb von 7,5 min (Eluent A: Natriumhydrogenphosphat 0,005 M, pH 7,4; Eluent B: Acetonitril/Natriumhydrogenphosphat 0,005 M, pH 7,4 (75/25); 2,0 ml/min. Die radiochemische Reinheit ist > 98 %.

### Beispiel 3 a

### N, N'-Bis-(2-Mercapto-1-(decyloxycarbonyl)-ethyl)-thiodiglycolsäurediamid

Zu einer Lösung von 2,98 g (10 mmol) Cysteindecylesterhydrochlorid (CA 57, 15235d) und 2,02 g (20 mmol) Triethylamin in 250 ml wasserfreiem Dichlormethan wird bei 0 °C und unter Argonatmosphäre eine Lösung von 0,935 g (5 mmol) Thiodiglycolsäuredichlorid, gelöst in 50 ml wasserfreiem Dichlormethan, getropft. Anschließend wird 1 h bei 0 °C und abschließend 16 h bei Raumtemperatur nachgerührt. Zur Aufarbeitung wäscht man die Dichlormethanlösung jeweils dreimal mit 2 %iger wäßriger Citronensäure, gesättigter Natriumhydrogencarbonatlösung und mit Wasser. Nach Trocknung über Natriumsulfat wird das Produkt durch Verdampfen des Lösungsmittels unter vermindertem Druck gewonnen. Zur Reinigung wird aus Diethylether kristallisiert.

Ausbeute: 2,57 g (80,7 %), weißes Pulver

| Analyse: | | | | | |
|---|---|---|---|---|---|
| Ber.: | C 56,57 | H 8,86 | N 4,40 | O 15,07 | S 15,10 |
| Gef.: | C 56,43 | H 8,92 | N 4,41 | | S 14,92 |

Eine alternative Darstellungsmethode für N,N'-Bis-(2-Mercapto-(decyloxycarbonyl)-ethyl)-thiodiglycolsäurediamid besteht darin, daß man 3,84 g (10 mmol) des unter Beispiel 1 a beschriebenen Liganden in 200 ml 1-Decanol unter Zugabe von 190 mg (1 mmol) Toluolsulfonsäurehydrat unter Argonatmosphäre 5 h auf 100 °C erhitzt, und dabei das gebildete Methanol abdestiliert. Nach beendeter Reaktion wird der Überschuß an 1-Decanol im Feinvakuum soweit wie möglich abdestilliert und der Rückstand in Dichlormethan aufgenommen. Die Dichlormethanlösung wird jeweils dreimal mit 2 %iger wäßriger Citronensäure, gesättigter Natriumhydrogencarbonatlösung und mit Wasser gewaschen. Nach Trocknung über Natriumsulfat wird unter vermindertem Druck das Lösungsmittel verdampft und der Rückstand an Kieselgel (Eluens: Dichlormethan/Methanol 99 : 1) chromatographiert. Abschließend kristallisiert man aus Diethylether.

Ausbeute: 843 mg (13,23 %), weißes Pulver

### Beispiel 3 b

### N,N'-Bis-(2-Mercapto-1-(decyloxycarbonyl)-ethyl)-thiodiglycolsäurediamid, Technetium-99m-Komplex

10 mg des unter Beispiel 3 a hergestellten Liganden werden in 1,0 ml Ethanol gelöst. 50 µl dieser Ligand-Lösung werden mit 250 µl Phosphat-Puffer pH 8,5, 50 µl einer desoxygenierten wäßrigen Citratlösung (50 mg/ml), 2,5 µl einer desoxygenierten wäßrigen Zinn(II)chlorid-Lösung (5 mg/ml 0,05 N HCl) und 100 µl einer Pertechnetat-Lösung (400 - 900 µCi) versetzt. Das Reaktionsgemisch wird nach einer Inkubationszeit von 10 min mittels HPLC auf die Reinheit des gebildeten Tc-Komplexes untersucht: Hamilton PRP-1 Säule, 5 µm, 125 x 4,6 mm; Gradientenelution von 100 % A nach 100 % B innerhalb von 7,5 min (Eluent A: Natriumhydrogenphosphat 0,005 M, pH 7,4; Eluent B: Acetonitril/Natriumhydrogen-phosphat 0,005 M, pH 7,4 (75/25); 2,0 ml/min. Die radiochemische Reinheit ist > 95 %.

### Beispiel 4 a

### N,N'-Bis-(2-Mercapto-1-(butylamino-carbonyl)-ethyl)-thiodiglycolsäurediamid

3,84 g (10 mmol) des unter Beispiel 1 a beschriebenen Liganden werden in 30 ml Ethanol und 70 ml n-Butylamin 6 h unter Argonatmosphäre zum Sieden erhitzt. Anschließend wird im Vakuum eingedämpft, der Rückstand mit 200 ml 2 %iger Citronensäure und 200 ml Dichlormethan unter Argonatmosphäre versetzt. Die Mischung wird 15 min heftig verrührt, die Dichlormethanphase abgetrennt und jeweils dreimal mit 2 %iger wäßriger Citronensäure, gesättigter Natriumhydrogencarbonatlösung und mit Wasser gewaschen. Nach Trocknung über Natriumsulfat verdampft man das Lösungsmittel unter vermindertem Druck und chromatographiert den Rückstand an Kieselgel (Eluens: Dichlormethan/Methanol 95 : 5). Abschließend wird das Reaktionsgemisch aus Diethylether umkristallisiert.

Ausbeute: 1,03 g (22,1 %), weißes Pulver

| Analyse: | | | | | |
|---|---|---|---|---|---|
| Ber.: | C 46,53 | H 6,94 | N 12,06 | O 13,77 | S 20,07 |
| Gef.: | C 46,37 | H 6,82 | N 11,89 | | S 19,78 |

### Beispiel 4 b

### N,N'-Bis-(2-Mercapto-1-(butylamino-carbonyl)-ethyl)-thiodiglycolsäurediamid, Technetium-99-Komplex

10 mg des unter Beispiel 4 a hergestellten Liganden werden in 1,0 ml Ethanol gelöst, 50 µl dieser Ligand-Lösung werden mit 250 µl Phosphat-Puffer pH 8,5, 50 µl einer desoxygenierten wäßrigen Citratlösung (50 mg/ml), 2,5 µl einer desoxygenierten wäßrigen Zinn(II)chlorid-Lösung (5mg/ml 0,05 N HCl) und 100 µl einer Pertechnetat-Lösung (400 - 900 µCi) versetzt. Das Reaktionsgemisch wird nach einer Inkubationszeit von 10 min mittels HPLC auf die Reinheit des gebildeten Tc-Komplexes untersucht: Hamilton PRP-1 Säule, 5 µm, 125 x 4,6 mm; Gradientenelution von 100 % A nach 100 % B innerhalb von 7,5 min (Eluent A: Natriumhydrogenphosphat 0,005 M, pH 7,4; Eluent B: Acetonitril/Natriumhydrogenphosphat 0,005 M, pH 7,4 (75/25); 2,0 ml/min. Die radiochemische Reinheit ist > 95 %.

### Beispiel 5 a

### N,N'-Bis-(2-Mercapto-1-(2-methoxyethoxy-carbonyl)-ethyl)-thiodiglycolsäurediamid

3,84 g (10 mmol) des unter Beispiel 1 a beschriebenen Liganden werden in Gegenwart von 190 mg (1 mmol) Toluolsulfonsäurehydrat unter Argonatmosphäre in 250 ml absolutem Ethylenglycolmonomethylether 6 h unter Rückfluß erhitzt. Anschließend verdampft man das Lösungsmittel unter vermindertem Druck und nimmt den öligen Rückstand in Dichlormethan auf. Die Dichlormethanlösung wird jeweils dreimal mit 2 %iger wäßriger Citronensäure, gesättigter Natriumhydrogencarbonatlösung und mit Wasser gewaschen. Nach Trocknung über Natriumsulfat verdampft man das Lösungsmittel unter vermindertem Druck und chromatographiert den öligen Rückstand an Kieselgel (Eluens: Dichlormethan/Methanol 8 : 2). Abschließend wird aus Diethylether kristallisiert.

Ausbeute: 753 mg (15,9 %), weißes Pulver

| Analyse: | | | | | |
|---|---|---|---|---|---|
| Ber.: | C 40,66 | H 5,97 | N 5,93 | O 27.08 | S 20,35 |
| Gef.: | C 40,37 | H 6,08 | N 5,71 | | S 20,08 |

### Beispiel 5 b

### N, N'-Bis-(2-Mercapto-1-(2-methoxyethoxy-carbonyl)-ethly)-thiodiglycolsäurediamid, Technetium-99m-Komplex

10 mg des unter Beispiel 5 a hergestellten Liganden werden in 1,0 ml Ethanol gelöst. 50 µl dieser Ligand-Lösung werden mit 250 µl Phosphat-Puffer pH 8,5, 50 µl einer desoxygenierten wäßrigen Citratlösung (50 mg/ml), 2,5 µl einer desoxygenierten wäßrigen Zinn(II)chlorid-Lösung (5 mg/ml 0,05 N HCl) und 100 µl einer Pertechnetat-Lösung (400 - 900 µCi) versetzt. Das Reaktionsgemisch wird nach einer Inkubationszeit von 10 min mittels HPLC auf die Reinheit des gebildeten Tc-Komplexes untersucht: Hamilton PRP-1 Säule, 5 µm. 125 x 4,6 mm; Gradientenelution von 100 % A nach 100 % B innerhalb von 7,5 min (Eluent A: Natriumhydrogenphosphat 0,005 M, pH 7,4; Eluent B: Acetonitril/Natriumhydrogenphosphat 0,005 M, pH 7,4 (75/25); 2,0 ml/min. Die radiochemische Reinheit ist > 95 %

### Beispiel 6 a

### N,N'-Bis-(2-Mercapto-1-(2,3-dihydroxypropylamino-carbonyl)-ethyl)-thiodiglycolsäurediamid

3,84 g (10 mmol) des unter Beispiel 1 a beschriebenen Liganden werden in 30 ml Ethanol und 30 ml Aminopropandiol 7 h unter Argonatmosphäre zum Sieden erhitzt. Anschließend wird unter vermindertem Druck das Ethanol abdestilliert, der Rückstand mit argongesättigtem Wasser versetzt und mit argongesättigter konzentrierter Salzsäure ein pH-Wert von 7 eingestellt. Die gelbliche Lösung wird gefriergetrocknet und der Rückstand an Kieselgel RP-18 (Eluens: Wasser/Tetrahydrofuran, Tetrahydrofuran 0 - 50 %) chromatographiert. Nach Verdampfen des Lösungsmittels erhält man ein farbloses Glas.

Ausbeute: 513 mg (10,2 %), farbloses Glas

| Analyse: bezogen auf die wasserfreie Substanz | | | | | |
|---|---|---|---|---|---|
| Ber.: | C 38,23 | H 6,02 | N 11,15 | O 25,47 | S 19,14 |
| Gef.: | C 38,11 | H 6,28 | N 10,88 | | S 18,95 |

### Beispiel 6 b

### N,N'-Bis-(2-Mercapto-1-(2,3-dihydroxypropylamino-carbonyl)-ethyl)-thiodiglycolsäurediamid, Technetium-99m-Komplex

10 mg des unter Beispiel 6 a hergestellten Liganden werden in 1,0 ml 50 % Ethanol gelöst. 50 µl dieser Ligand-Lösung werden mit 250 µl Phosphat-Puffer pH 8,5, 50 µl einer desoxygenierten wäßrigen Citratlösung 50 mg/ml), 2,5 µl einer desoxygenierten wäßrigen Zinn(II)chorid-Lösung (5 mg/ml 0,05 N HCl) und 100 µl einer Pertechnetat-Lösung (400 - 900 µCi) versetzt. Das Reaktionsgemisch wird nach einer Inkubationszeit von 10 min mittels HPLC auf die Reinheit des gebildeten Tc-Komplexes untersucht: Hamilton PRP-1 Säule, 5 µm, 125 x 4,6 mm; Gradienteneluation von 100 % A nach 100 % B innerhalb von 7,5 min (Eluent A: Natriumhydrogenphosphat 0,005 M, pH 7,4; Eluent B: Acetonitril/Natriumhydrogenphosphat 0,005 M, pH 7,4 (75/25); 2,0 ml/min. Die radiochemische Reinheit ist > 95 %.

### Beispiel 7 a

### N-(4-Thia-1-(ethoxy-carbonyl)-pentyl)-thiodiglycolsäuremonoamid

Zu einer Lösung aus 21,37 g (0,1 mol) Methioninethylesterhydrochlorid und 20,24 g (0,2 mol) Triethylamin in 1 l wasserfreiem Dichlormethan tropft man unter Argonatmosphäre bei 0 °C 13,21 g (0,1 mol) Thiodiglycolsäureanhydrid, gelöst in 500 ml wasserfreiem Dichlormethan. Anschließend wird 1 h bei 0 °C und abschließend 16 h bei Raumtemperatur nachgerührt. Zur Aufarbeitung versetzt man mit 2 %iger wäßriger Citronensäure, rührt gut durch und stellt durch tropfenweise Zugabe von konzentrierter Salzsäure pH = 2 ein. Die organische Phase wird abgetrennt und über Natriumsulfat getrocknet. Nach Verdampfen des Lösungsmittels erhält man ein gelbliches Öl, das ohne weitere Reinigung für weitere Umsetzungen benutzt werden kann.

Ausbeute: 25,74 g (83,2 %), gelbliches Öl

| Analyse: | | | | | |
|---|---|---|---|---|---|
| Ber.: | C 42,70 | H 6,19 | N 4,53 | O 25,86 | S 20,72 |
| Gef.: | C 42,37 | H 6,62 | N 4,15 | | S 20,31 |

### Beispiel 7 b

### N-(4-Thia-1-(ethoxy-carbonyl)-pentyl-N'-(2-oxo-tetrahydrothiophen-3-yl)-thiodiglycolsäurediamid

Zu einer Lösung aus 3,09 g (10 mmol) des unter Beispiel 7 a hergestellten Thiodiglycolsäuremonamid-Derivates und 2,02 g (20 mmol) Triethylamin in 100 ml wasserfreiem Dichlormethan tropft man bei - 15 °C eine Lösung aus 1,09 g (10 mmol) Chlorameisensäureethylester in 25 ml wasserfreiem Dichlormethan. Nach beendeter Aktivierung (45 min) wird eine Lösung aus 1,54 g (10 mmol) Homocysteinthiolactonhydrochlorid und 2,02 g (10 mmol) Triethylamin in 25 ml wasserfreiem Dichlormethan zugetropft. Man rührt 1 h bei - 15 °C und läßt über Nacht auf Raumtemperatur kommen. Zur Aufarbeitung wäscht man die Dichlormethanlösung jeweils dreimal mit 2 %iger wäßriger Citronensäure, gesättigter Natriumhydrogencarbonatlösung und mit Wasser. Nach Trocknung über Natriumsulfat wird unter vermindertem Druck vom Lösungsmittel entfernt und der verbleibende Rückstand aus Diethylether kristallisiert.

Ausbeute: 3,75 g (91,8 %), weißes Pulver

| Analyse: | | | | | |
|---|---|---|---|---|---|
| Ber.: | C 44,10 | H 5,92 | N 6,86 | O 19,58 | S 23,54 |
| Gef.: | C 43,97 | H 5,98 | N 6,72 | | S 23,31 |

### Beispiel 7 c

### N-(4-Thia-1-(ethoxy-carbonyl)-pentyl)-N'-(3-mercapto-1-(butylaminocarbonyl)-propyl)-thiodiglycolsäurediamid

Zu einer Lösung aus 2,04 g (5 mmol) des unter Beispiel 7 b hergestellten Thiolacton-Derivates des Thiodiglycolsäurediamids in 20 ml wasserfreiem Dichlormethan gibt man unter Argonatmosphäre 0,731 g (10 mmol) Butylamin. Man läßt über Nacht bei Raumtemperatur rühren und dampft das Reaktionsgemisch unter vermindertem Druck ein. Anschließend versetzt man mit 2 %iger wäßriger Citronensäure und Dichlormethan und rührt gut durch. Nach Abtrennung der organischen Phase wird diese jeweils dreimal mit 2 %iger wäßriger Citronensäure, gesättigter Natriumhydrogencarbonatlösung und mit Wasser gewaschen. Nach Trocknung über Natriumsulfat wird das Lösungsmittel unter vermindertem Druck verdampft. Der Rückstand kann zur Reinigung an Kieselgel (Eluens: Dichlormethan/Methanol 95:5) chromatographiert werden.

Ausbeute: 1,51 g (62,7 %), weißes Pulver

| Analyse: | | | | | |
|---|---|---|---|---|---|
| Ber.: | C 47,38 | H 7,32 | N 8,72 | O 16,61 | S 19,97 |
| Gef.: | C 47,23 | H 7,41 | N 8,62 | | S 19,79 |

### Beispiel 7 d

### N-(4-Thia-1-(ethoxy-carbonyl)-pentyl)-N'-(3-mercapto-1-(butylaminocarbonyl)-propyl)-thiodiglycolsäurediamid, Technetium-99m-Komplex

10 mg des unter Beispiel 7 c hergestellten Liganden werden in 1,0 ml Ethanol gelöst. 50 µl dieser Ligand-Lösung werden mit 250 µl Phosphat-Puffer pH 8,5, 50 µl einer desoxygenierten wäßrigen Citratlösung (50 mg/ml), 2,5 µl einer desoxygenierten wäßrigen Zinn(II)chlorid-Lösung (5 mg/ml 0,05 N HCl) und 100 µl einer Pertechnetat-Lösung (400 - 900 µCi) versetzt. Das Reaktionsgemisch wird nach einer Inkubationszeit von 10 min mittels HPLC auf die Reinheit des gebildeten Tc-Komplexes untersucht: Hamilton PRP-1 Säule, 5 µm, 125 x 4,6 mm; Gradientenelution von 100 % A nach 100 % B innerhalb von 7,5 min (Eluent A: Natriumhydrogenphosphat 0,005 M, pH 7,4; Eluent B: Acetonitril/Natriumhydrogenphosphat 0,005 M, pH 7,4 (75/25); 2,0 ml/min. Die radiochemische Reinheit ist > 95 %.

### Beispiel 8 a

### N-(4-Thia-1-(hydroxy-carbonyl)-pentyl)-N'-(3-mercapto-1-(hydroxy-carbonyl)-propyl)-thiodiglycolsäurediamid

Unter Argonatmosphäre werden 2,04 g (5 mmol) des unter Beispiel 7 b hergestellten Thiolacton-Derivates des Thiodiglycolsäurediamids in 500 ml 2 N wäßriger Natriumhydroxidlösung mit 50 ml Ethanol in Lösung gebracht. Man rührt 1 h bei Raumtemperatur, stellt mit argongesättigter, konzentrierter Salzsäure pH = 2 ein und extrahiert das ausgeschiedene Öl mit argongesättigtem Essigsäureethylester. Nach Trocknung der organischen Phase über Natriumsulfat wird das Lösungsmittel unter vermindertem Druck verdampft und das ölige Rohprodukt durch Verreiben mit Diethylether kristallisiert.

Ausbeute: 666 mg (33,4 %), weißes Pulver

| Analyse: bezogen auf die wasserfreie Substanz | | | | | |
|---|---|---|---|---|---|
| Ber.: | C 39,18 | H 5,56 | N 7,03 | O 24,09 | S 24,13 |
| Gef.: | C 39,02 | H 5,79 | N 6,84 | | S 23,83 |

### Beispiel 8 b

### N-(4-Thia-1-(hydroxy-carbonyl)-pentyl)-N'-(3-mercapto-1-(hydroxy-carbonyl)-propyl)-thiodiglycolsäurediamid, Technetium-99m-Komplex

10 mg des unter Beispiel 8 a hergestellten Liganden werden in 1,0 ml 0,5 M Phosphat-Puffer pH 7,5 gelöst. 50 µl dieser Ligand-Lösung werden mit 250 µl Phosphat-Puffer pH 8,5, 50 µl einer desoxygenierten wäßrigen Citratlösung (50 mg/ml), 2,5 µl einer desoxygenierten wäßrigen Zinn(II)chlorid-Lösung (5 mg/ml 0,05 N HCl) und 100 µl einer Pertechnetat-Lösung (400 - 900 µCi) versetzt. Das Reaktionsgemisch wird nach einer Inkubationszeit von 10 min mittels HPLC auf die Reinheit des gebildeten Tc-Komplexes untersucht: Hamilton PRP-1-Säule, 5 µm, 125 x 4,6 mm; Gradientenelution von 100 % A nach 100 % B innerhalb von 7,5 min (Eluent A: Natriumhydrogenphosphat 0,005 M, pH 7,4; Eluent B: Acetoninitril/Natriumhydrogenphosphat 0,005 M, pH 7,4 (75/25); 2,0 ml/min: Die radiochemische Reinheit ist > 95 %.

### Beispiel 9 a

### N-(3-Mercapto-1-(carbonyl-Gly-His-Leu-Asp-Ile-Ile-Trp)-propyl)-N'-(4-thia-1-(ethoxycarbonyl)-pentyl)-thiodiglycolsäurediamid

Zu einer Lösung von 853 mg (1 mmol) NH₂-Gly-His-Leu-Asp-Ile-Ile-Trp (hergestellt in Analogie zu Barany und Merrifield, The Peptides: Analysis, Biology, Academic Press, New York, 1980; Stewart und Young, Solid Phase Peptides Syntheses, 2^{nd}ed., Pierce Chemical W., Rockford, II, 1984) und 304 mg (3 mmol) Triethylamin in 100 ml wasserfreiem Dimethylformamid unter Argonatmosphäre gibt man 408,5 mg (1 mmol) des unter 7 b hergestellten N-(4-Thia-1-(ethoxy-carbonyl)-pentyl)-N'-(2-oxo-tetrahydrothiophen-3-yl)-thiodigylcolsäurediamid und rührt das resultierende Reaktionsgemiisch 14 h bei Raumtemperatur. Nach beendeter Reaktion wird filtriert und das Lösungsmittel unter vermindertem Druck abgezogen. Das verbleibende Öl wird dreimal mit 50 ml Dimethylformamid versetzt und jeweils eingedampft. Man verrührt den Rückstand mit 200 ml wasserfreiem Diethylether, worauf sich ein weißer Feststoff abscheidet, der abfiltriert wird. Zur Reinigung wird aus Dimethylformamid/Diethylethergemischen umkristallisiert.

Ausbeute: 345 mg (27,3 %), weißes Pulver

| Analyse: bezogen auf die wasserfreie Substanz | | | | | |
|---|---|---|---|---|---|
| Ber.: | C 53,32 | H 6,71 | N 13,32 | O 19,02 | S 7,62 |
| Gef.: | C 53,17 | H 6,83 | N 13,19 | | S 7,42 |

### Beispiel 9 b

### N-(3-Mercapto-1-(carbonyl-Gly-His-Leu-Asp-Ile-Ile-Trp)-propyl)-N'-(4-thia-1-(ethoxycarbonyl)-pentyl)-thiodiglycolsäurediamid, Technetium-99m-Kompex

10 mg des unter Beispiel 9 a hergestellten Liganden werden in 1,0 ml 0,5 M Phosphat-Puffer pH 7,5 gelöst. 50 µl dieser Ligand-Lösung werden mit 250 µl Phosphat-Puffer pH 8,5, 50 µl einer desoxygenierten wässrigen Citratlösung (50 mg/ml), 2,5 µl einer desoxygenierten wässrigen Zinn(II)chlorid-Lösung (5 mg/ml 0,05 N HCl) und 100 µl einer Pertechnetat-Lösung (400 - 900 µCi) versetzt. Das Reaktionsgemisch wird nach einer Inkubationszeit von 10 min mittels HPLC auf die Reinheit des gebildeten Tc-Komplexes untersucht: Hamilton PRP-1 Säule, 5 µm, 125 x 4,6 mm; Gradientenelution von 100 % A nach 100 % B innerhalb von 7,5 min (Eluent A: Natriumhydrogenphosphat 0,005 M, pH 7,4; Eluent B: Acetonitril/Natriumhydrogenphosphat 0,005 M, pH 7,4 (75/25); 2,0 ml/min. Die radiochemische Reinheit ist >96 %.

### Beispiel 10 a

### N-(3-Mercapto-1-(carbonyl-Gly-His-Leu-Asp-Ile-Ile-Trp)-propyl)-N'-(4-thia-1-(hydroxy-carbonyl)-pentyl)-thiodiglycolsäurediamid

126 mg (0,1 mmol) des unter Beispiel 9 a hergestellten N-(3-Mercapto-1-(carbonyl-Gly-His-Leu-Asp-Ile-Ile-Trp)-propyl)-N'-(4-thia-1-(ethoxycarbonyl)-pentyl)-thiodiglycolsäurediamid werden unter Argon in 50 ml 0,1 N wässriger Natriumhydroxidlösung gelöst. Man rührt 1 h bei Raumtemperatur, stellt mit 1 N wässriger Salzsäure einen pH-Wert von 7 ein und isoliert das Rohprodukt durch Gefriertrocknung. Zur Reinigung wird an Kieselgel RP-18 (Eluens: Wasser/Tetrahydrofuran; Tetrahydrofuran: 0 - 30 %) chromatographiert. Die die Zielverbindung enthaltenden Fraktionen werden nach Abdestilieren des Tetrahydrofurans gefriergetrocknet. Man löst den Rückstand in wenig Wasser, stellt durch Zugabe von wässriger Salzsäure eine pH-Wert von 2 ein und filtriert das Produkt ab.

Ausbeute: 23 mg (18,6 %), weißes Pulver

| Analyse: bezogen auf die wasserfreie Substanz | | | | | |
|---|---|---|---|---|---|
| Ber.: | C 52,58 | H 6,54 | N 13,63 | O 19,46 | S 8,00 |
| Gef.: | C 52,37 | H 6,79 | N 13,41 | | S 7,77 |

### Beispiel 10 b

### N-(3-Mercapto-1-(carbonyl-Gly-His-Leu-Asp-Ile-Ile-Trp)-propyl)-N'-(4-thia-1-(hydroxy-carbonyl)-pentyl)-thiodiglycolsäurediamid, Technetium-99m-Komplex

10 mg des unter Beispiel 10 a hergestellten Liganden werden in 1,0 ml 0,5 M Phosphat-Puffer pH 7,5 gelöst. 50 µl dieser Ligand-Lösung werden mit 250 µl Phosphat-Puffer pH 8,5, 50 µl einer desoxygenierten wässrigen Ci-tratlösung (50 mg/ml), 2,5 µl einer desoxygenierten wässrigen Zinn(II)chlorid-Lösung (5 mg/ml 0,05 N HCl) und 100 µl einer Pertechnetat-Lösung (400 - 900 µCi) versetzt. Das Reaktionsgemisch wird nach einer Inkubationszeit von 10 min mittels HPLC auf die Reinheit des gebildeten Tc-Komplexes untersucht: Hamilton PRP-1 Säule, 5 µm, 125 x 4,6 mm; Gradientenelution von 100 % A nach 100 % B innerhalb von 7,5 min (Eluent A: Natriumhydrogenphosphat 0,005 M, pH 7,4; Eluent B: Acetonitril/Natriumhydrogenphosphat 0,005 M, pH 7,4 (75/25); 2,0 ml/min. Die radiochemische Reinheit ist > 95 %.

### Beispiel 11

### Anreicherung von N,N'-Bis-(2-Mercapto-1-(decyloxycarbonyl)-ethyl)-thiodiglycolsäurediamid, Technetium-99m-Komplex in atherosklerotischen Gefäßläsionen von WHHL-Kaninchen

Die Markierung des N,N'-Bis-(2-Mercapto-1-(decyloxycarbonyl)-ethyl)-thiodiglycolsäurediamids (hergestellt nach Beispiel 3 a) erfolgt wie in Beispiel 3 b beschrieben.

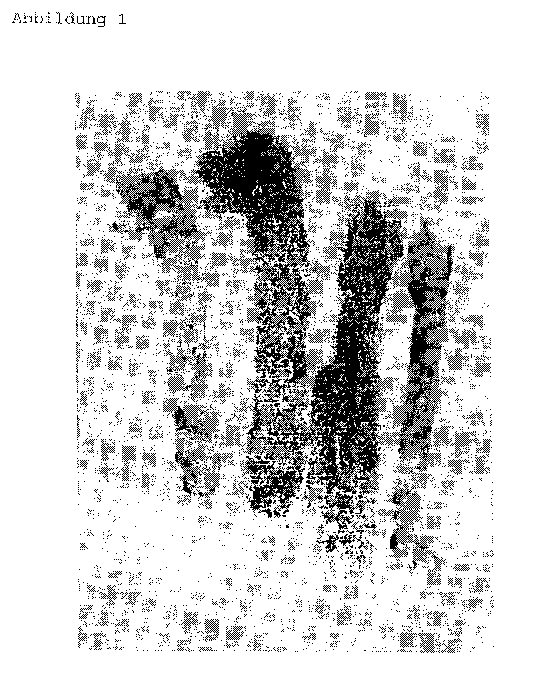

99,9 GBq (2,7 mCi) der nach Beispiel 3 b markierten Substanz wurde mit phosphatgepufferter Saline auf 1 ml verdünnt und einem narkotisierten WHHL-Kaninchen Rompun/Ketavet (1:2) über eine Ohrvene appliziert. 5 h nach Applikation wurde das Kaninchen getötet und sowohl eine Autoradiographie der Aorta als auch eine Sudan-III-Färbung zur Darstellung der atherosklerotischen Plaques durchgeführt (Abbildung 1). Der Anreicherungsfaktor zwischen normalen und atherosklerotischen Wandbereichen betrug je nach Ausbildung der Plaques (Sudan III-Färbung) zwischen 3 und 8.

## Patentansprüche

1. Verbindungen der allgemeinen Formel (I)
B-CO-CR¹R²-A-CR³R⁴-CO-D (I)
worin
A für ein Chalkogenatom O, S oder Se steht,
R¹, R², R³ und R⁴ gleich oder unterschiedlich sind und jeweils für ein Wasserstoffatom und/oder für einen verzweigten oder unverzweigten C₁-C₆-Alkylrest stehen,
B und D gleich oder unterschiedlich sind und einen Rest
-NH-CR⁵R⁶-(CR⁷R⁸)_{n=1,2}-S-R⁹
darstellen, worin
R⁵ und R⁶ gleich oder unterschiedlich sind und jeweils ein Wasserstoffatom oder einen unverzweigten, verzweigten, cyclischen oder polycyclischen C₁-C₆₀-Alkyl-, Alkenyl-, Polyalkenyl-, Alkinyl-, Polyalkinyl-, Aryl-, Alkylaryl- oder Arylalkyl-Rest darstellen, welcher gegebenenfalls mit Hydroxy-, Carboxy-, Aminocarbonyl-, Alkoxycarbonyl-, Amino-, Aldehyd-, Oxo-, Oxy- oder Alkoxy-Gruppen mit bis zu 20 Kohlenstoffatomen substituiert ist und/oder gegebenenfalls durch ein oder mehrere Heteroatome aus der Reihe O, N, S, P, As, Se unterbrochen und/oder substituiert ist,
R⁷ und R⁸ gleich oder unterschiedlich sind und jeweils für ein Wasserstoffatom und/oder für einen verzweigten oder unverzweigten C₁-C₆-Alkylrest stehen,
R⁹ für ein Wasserstoffatom, für einen verzweigten oder unverzweigten C₁-C₆-Alkylrest oder für eine Schwefelschutzgruppe steht und
R⁹ und R⁵ gegebenenfalls zusammen mit den sie verbindenden Gruppen einen gegebenenfalls mit Hydroxy-, Oxo-, Oxy- oder Alkoxy-Gruppen mit bis zu 6 Kohlenstoffatomen substituierten 4- bis 8-gliedrigen Ring bilden,
deren Konjugate mit sich selektiv in erkranktem Gewebe anreichernde Substanzen, wobei zwischen diesen eine kovalente Bindung besteht und diese im Falle von Carboxy- oder Aminogruppen enthaltenden Substanzen wie Peptiden, Proteinen und Antikörpern oder deren Fragmenten, amidisch oder im Falle von Hydroxygruppen enthaltenden Substanzen wie Fettalkoholen, esterartig oder im Falle von Aldehydgruppen enthaltenden Substanzen imidisch vorliegt
sowie deren Komplexe mit Radioisotopen von Tc oder Re.

2. Verbindungen nach Anspruch 1, dadurch gekennzeichnet, daß R¹, R², R³, R⁴ und R⁵ Wasserstoffatome darstellen.

3. Verbindungen nach Anspruch 1, dadurch gekennzeichnet, daß R¹, R², R³, R⁴, R⁵' R⁷ und R⁸ Wasserstoffatome sind.

4. Verbindungen nach mindestens einem der Ansprüche 1 bis 3, dadurch gekennzeichnet, daß die sich in erkranktem Gewebe anreichernden Substanzen Peptide wie Endotheline, Teilsequenzen von Endothelinen, Endothelin-Analoga, Endothelin-Derivate oder Endothelin-Antagonisten bedeuten.

5. Verbindungen nach mindestens einem der Ansprüche 1 bis 4, dadurch gekennzeichnet, daß die Peptide die folgenden Sequenzen oder Teile davon aufweisen 
Cys-Ser-Cys-Ser-Ser-Leu-Met-Asp-Lys-Glu-Cys-Val-Tyr-
Phe-Cys-His-Leu-Asp-Ile-Ile-Trp,

Cys-Ser-Cys-Ser-Ser-Trp-Leu-Asp-Lys-Glu-Cys-Val-Tyr-
Phe-Cys-His-Leu-Asp-Ile-Ile-Trp,

Cys-Thr-Cys-Phe-Thr-Tyr-Lys-Asp-Lys-Glu-Cys-Val-Tyr-
Tyr-Cys-His-Leu-Asp-Ile-Ile-Trp,

Cys-Ser-Ala-Ser-Ser-Leu-Met-Asp-Lys-Glu-Ala-Val-Tyr-
Phe-Cys-His-Leu-Asp-Ile-Ile-Trp,
 
Cys-Ser-Cys-Asn-Ser-Trp-Leu-Asp-Lys-Glu-Cys-Val-Tyr-
Phe-Cys-His-Leu-Asp-Ile-Ile-Trp,

Cys-Ser-Cys-Lys-Asp-Met-Thr-Asp-Lys-Glu-Cys-Leu-Asn-
Phe-Cys-His-Gln-Asp-Val-Ile-Trp,

Ala-Ser-Cys-Ser-Ser-Leu-Met-Asp-Lys-Glu-Cys-Val-Tyr-
Phe-Ala-His-Leu-Asp-Ile-Ile-Trp,

Ala-Ser-Ala-Ser-Ser-Leu-Met-Asp-Lys-Glu-Ala-Val-Tyr-
Phe-Ala-His-Leu-Asp-Ile-Ile-Trp,

Cys-Ser-Cys-Ser-Ser-Trp-Leu-Asp-Lys-Glu-Ala-Val-Tyr-
Phe-Ala-His-Leu-Asp-Ile-Ile-Trp,

Cys-Val-Tyr-Phe-Cys-His-Leu-Asp-Ile-Ile-Trp,

N-Acetyl-Leu-Met-Asp-Lys-Glu-Ala-Val-Tyr-Phe-Ala-
His-Leu-Asp-Ile-Ile-Trp,
 die Teilsequenz
His-Leu-Asp-Ile-Ile-Trp
oder die cyclischen Aminosäuresequenzen
Cyclo-(DTrp-DAsp-Pro-DVal-Leu),
Cyclo-(DGlu-Ala-alloDIle-Leu-DTrp)
aufweisen.

6. Verfahren zur Herstellung der Verbindungen nach Anspruch 1, dadurch gekennzeichnet, daß man Verbindungen der allgemeinen Formel (II)
X-CO-CR¹R²-A-CR³R⁴-CO-X' (II)
worin R¹, R², R³, R⁴, und A die im Anspruch 1 angegebene Bedeutung haben und
X, X' für eine Abgangsgruppe stehen mit Verbindungen der allgemeinen Formel (III)
H-B (III)
und/oder der allgemeinen Formel (IV)
H-D (IV)
umsetzt,
wobei B und D die in Anspruch 1 gegebene Bedeutung haben,
und daß
gegebenenfalls die so hergestellten Verbindungen mit sich selektiv in erkranktem Gewebe oder in Tumoren anreichernden Substanzen konjugiert werden, wobei zwischen diesen eine kovalente Bindung geknüpft wird, welche im Falle von Aminogruppen enthaltenden Substanzen wie Peptiden, Proteinen und Antikörpern, amidisch oder im Falle von Hydroxygruppen enthaltenden Substanzen wie Alkoholen, esterartig oder im Falle von Aldehydgruppen enthaltenden Substanzen imidisch erfolgt,
und daß
die so hergestellten Verbindungen und Konjugate mit Technetium-99m oder Re in Form von Pertechnetat oder Perrhenat in Gegenwart eines Reduktionsmittels und gegebenenfalls eines Hilfsliganden umgesetzt werden.

7. Radiopharmazeutische Zusammensetzung zur nicht invasiven in-vivo-Darstellung von Rezeptoren und rezeptorhaltigem Gewebe und/oder von atherosklerotischen Plaques, dadurch gekennzeichnet, daß sie eine Verbindung oder ein Konjugat gemäß einem der Ansprüche 1 bis 5, sowie gegebenenfalls mit den in der Galenik üblichen Zusätzen, enthält.

## Claims

1. Compounds of the general formula (I)
B-CO-CR¹R²-A-CR³R⁴-CO-D (I),
wherein
A represents a chalcogen atom O, S or Se,
R¹, R², R³ and R⁴ are the same or different and each represents a hydrogen atom and/or a branched or unbranched C₁-C₆alkyl radical,
B and D are the same or different and represent a radical
-NH-CR⁵R⁶-(CR⁷R⁸)_{n=1,2}-S-R⁹,
wherein
R⁵ and R⁶ are the same or different and each represents a hydrogen atom or an unbranched or branched, cyclic or polycyclic C₁-C₆₀-alkyl, -alkenyl, -polyalkenyl, -alkynyl, -polyalkynyl, -aryl, -alkylaryl or -arylalkyl radical, which is optionally substituted by hydroxy, carboxy, aminocarbonyl, alkoxycarbonyl, amino, aldehyde, oxo, oxy or alkoxy group(s) having up to 20 carbon atoms and/or optionally interrupted and/or substituted by one or more hetero atoms from the series O, N, S, P, As and Se,
R⁷ and R⁸ are the same or different and each represents a hydrogen atom and/or a branched or unbranched C₁-C₆alkyl radical,
R⁹ represents a hydrogen atom, a branched or unbranched C₁-C₆alkyl radical or a sulphur-protecting group, and
R⁹ and R⁵ optionally together with the groups connecting them form a 4- to 8-membered ring optionally substituted by hydroxy, oxo, oxy or alkoxy group(s) having up to 6 carbon atoms,
conjugates thereof with substances that accumulate selectively in diseased tissue, wherein the compounds and those substances are covalently bonded, the covalent bond being an amide bond in the case of substances containing carboxy or amino groups, such as peptides, proteins and antibodies or fragments thereof, or an ester-type bond in the case of substances containing hydroxy groups, such as fatty alcohols, or an imide bond in the case of substances containing aldehyde groups,
and complexes thereof with radioisotopes of Tc or Re.

2. Compounds according to claim 1, characterised in that R¹, R², R³, R⁴ and R⁵ represent hydrogen atoms.

3. Compounds according to claim 1, characterised in that R¹, R², R³, R⁴, R⁵, R⁷ and R⁸ represent hydrogen atoms.

4. Compounds according to at least one of claims 1 to 3, characterised in that the substances that accumulate in diseased tissue are peptides, such as endothelins, endothelin partial sequences, endothelin analogues, endothelin derivatives or endothelin antagonists.

5. Compounds according to at least one of claims 1 to 4, characterised in that the peptides have the following sequences or portions thereof 
Cys-Ser-Cys-Ser-Ser-Leu-Met-Asp-Lys-Glu-Cys-Val-Tyr-
Phe-Cys-His-Leu-Asp-Ile-Ile-Trp,
Cys-Ser-Cys-Ser-Ser-Trp-Leu-Asp-Lys-Glu-Cys-Val-Tyr-
Phe-Cys-His-Leu-Asp-Ile-Ile-Trp,
 
Cys-Thr-Cys-Phe-Thr-Tyr-Lys-Asp-Lys-Glu-Cys-Val-Tyr-
Tyr-Cys-His-Leu-Asp-Ile-Ile-Trp,

Cys-Ser-Ala-Ser-Ser-Leu-Met-Asp-Lys-Glu-Ala-Val-Tyr-
Phe-Cys-His-Leu-Asp-Ile-Ile-Trp,

Cys-Ser-Cys-Asn-Ser-Trp-Leu-Asp-Lys-Glu-Cys-Val-Tyr-
Phe-Cys-His-Leu-Asp-Ile-Ile-Trp,

Cys-Ser-Cys-Lys-Asp-Met-Thr-Asp-Lys-Glu-Cys-Leu-Asn-
Phe-Cys-His-Gln-Asp-Val-Ile-Trp,

Ala-Ser-Cys-Ser-Ser-L[?]u-Met-Asp-Lys-Glu-Cys-Val-Tyr-
Phe-Ala-His-Leu-Asp-I[?]e-Ile-Trp,

Ala-Ser-Ala-Ser-Ser-Leu-Met-Asp-Lys-Glu-Ala-Val-Tyr-
Phe-Ala-His-Leu-Asp-Ile-Ile-Trp,

Cys-Ser-Cys-Ser-Ser-Trp-Leu-Asp-Lys-Glu-Ala-Val-Tyr-
Phe-Ala-His-Leu-Asp-Ile-Ile-Trp,

Cys-Val-Tyr-Phe-Cys-His-Leu-Asp-Ile-Ile-Trp,

N-acetyl-Leu-Met-Asp-Lys-Glu-Ala-Val-Tyr-Phe-Ala-
His-Leu-Asp-Ile-Ile-Trp,
 the partial sequence
His-Leu-Asp-Ile-Ile-Trp
or the cyclic amino acid sequences
cyclo-(DTrp-DAsp-Pro-DVal-Leu),
cyclo-(DGlu-Ala-alloDIle-Leu-DTrp).

6. Process for the preparation of the compounds according to claim 1, characterised in that compounds of the general formula (II)
X-CO-CR¹R²-A-CR³R⁴-CO-X' (II),
wherein R¹, R², R³, R⁴ and A have the meanings given in claim 1 and X and X' represent a leaving group,
are reacted with compounds of the general formula (III)
H-B (III)
and/or of the general formula (IV)
H-D (IV),
wherein B and D have the meanings given in claim 1,
and
optionally the compounds so prepared are conjugated with substances that accumulate selectively in diseased tissue or in tumours, wherein the compounds and those substances are covalently bonded, the covalent bond being an amide bond in the case of substances containing amino groups, such as peptides, proteins and antibodies, or an ester-type bond in the case of substances containing hydroxy groups, such as alcohols, or an imide bond in the case of substances containing aldehyde groups
and
the compounds and conjugates so prepared are reacted with technetium-99m or Re in the form of pertechnetate or perrhenate in the presence of a reducing agent and, optionally, an auxiliary ligand.

7. Radiopharmaceutical composition for non-invasive *in vivo* visualisation of receptors and receptor-containing tissue and/or of atherosclerotic plaques, characterised in that it comprises a compound or a conjugate according to any one of claims 1 to 5, optionally together with the additives customary in galenical pharmacy.

## Revendications

1. Composés répondant à la formule générale (I)
B-CO-CR¹R²-A-CR³ R⁴-CO-D (I)
dans laquelle A représente un atome de chalcogène O, S ou Se,
R¹, R², R³ et R⁴ sont identiques ou différents et représentent chacun un atome d'hydrogène et/ou un radical alkyle en C₁-C₆ ramifié ou non ramifié,
B et D sont identiques ou différents et représentent un radical
-NH-CR⁵R⁶-(CR⁷R⁸)_{n=1,2}-S-R⁹,
où
R⁵ et R⁶ sont identiques ou différents et représentent chacun un atome d'hydrogène ou un radical arylalkyle, alkylaryle, aryle, polyalcynyle, alcynyle, polyalcényle, alcényle ou alkyle en C₁-C₆₀ cyclique ou polycyclique, ramifié ou non ramifié, qui est éventuellement substitué par des groupes hydroxy, carboxy, aminocarbonyle, alcoxycarbonyle, amino, aldéhyde, oxo, oxy ou alcoxy comportant jusqu'à 20 atomes de carbone et est éventuellement interrompu et/ou substitué par un ou plusieurs hétéroatomes de la série O, N, S, P, As, Se,
R⁷ et R⁸ sont identiques ou différents et représentent chacun un atome d'hydrogène et/ou un radical alkyle en C₁-C₆ ramifié ou non ramifié,
R⁹ représente un atome d'hydrogène, un radical alkyle en C₁-C₆ ramifié ou non ramifié ou un groupe de protection de soufre, et
R⁹ et R⁵ forment éventuellement conjointement avec les groupes qui les relient un noyau rectangulaire à octogonal éventuellement substitué par des groupes hydroxy, oxo, oxy ou alcoxy comportant jusqu'à 6 atomes de carbone,
leurs produits de conjugaison avec des substances qui s'enrichissent de manière sélective dans un tissu atteint de maladie, une liaison covalente existant entre ces derniers et celle-ci étant de type amide dans le cas de substances contenant des groupes carboxy ou amino, comme des peptides, des protéines et des anticorps ou leurs fragments, ou de type ester, dans le cas de substances contenant des groupes hydroxy, comme des alcools gras, ou de type imide, dans le cas de substances contenant des groupes aldéhyde,
ainsi que leurs complexes avec des radioisotopes de Tc ou de Re.

2. Composés suivant la revendication 1, caractérisés en ce que R¹, R², R³, R⁴ et R⁵ représentent des atomes d'hydrogène.

3. Composés suivant la revendication 1, caractérisés en ce que R¹, R², R³, R⁴, R⁵, R⁷ et R⁸ sont des atomes d'hydrogène.

4. Composés suivant au moins l'une des revendications 1 à 3, caractérisés en ce que les substances qui s'enrichissent dans le tissu atteint de maladie représentent des peptides, tels que des endothélines, des séquences partielles d'endothélines, des analogues d'endothélines, des dérivés d'endothélines ou des antagonistes d'endothélines.

5. Composés suivant au moins l'une des revendications 1 à 4, caractérisés en ce que les peptides présentent les séquences suivantes ou des parties de celles-ci 
Cys-Ser-Cys-Ser-Ser-Leu-Met-Asp-Lys-Glu-Cys-Val-Tyr-
Phe-Cys-His-Leu-Asp-Ile-Ile-Trp,

Cys-Ser-Cys-Ser-Ser-Trp-Leu-Asp-Lys-Glu-Cys-Val-Tyr-
Phe-Cys-His-Leu-Asp-Ile-Ile-Trp,
 
Cys-Thr-Cys-Phe-Thr-Tyr-Lys-Asp-Lys-Glu-Cys-Val-Tyr-
Tyr-Cys-His-Leu-Asp-Ile-Ile-Trp,

Cys-Ser-Ala-Ser-Ser-Leu-Met-Asp-Lys-Glu-Ala-Val-Tyr-
Phe-Cys-His-Leu-Asp-Ile-Ile-Trp,

Cys-Ser-Cys-Asn-Ser-Trp-Leu-Asp-Lys-Glu-Cys-Val-Tyr-
Phe-Cys-His-Leu-Asp-Ile-Ile-Trp,

Cys-Ser-Cys-Lys-Asp-Met-Thr-Asp-Lys-Glu-Cys-Leu-Asn-
Phe-Cys-His-Gln-Asp-Val-Ile-Trp,

Ala-Ser-Cys-Ser-Ser-Leu-Met-Asp-Lys-Glu-Cys-Val-Tyr-
Phe-Ala-His-Leu-Asp-Ile-Ile-Trp,

Ala-Ser-Ala-Ser-Ser-Leu-Met-Asp-Lys-Glu-Ala-Val-Tyr-
Phe-Ala-His-Leu-Asp-Ile-Ile-Trp,

Cys-Ser-Cys-Ser-Ser-Trp-Leu-Asp-Lys-Glu-Ala-Val-Tyr-
Phe-Ala-His-Leu-Asp-Ile-Ile-Trp,

Cys-Val-Tyr-Phe-Cys-His-Leu-Asp-Ile-Ile-Trp,

N-Acetyl-Leu-Met-Asp-Lys-Glu-Ala-Val-Tyr-Phe-Ala-
His-Leu-Asp-Ile-Ile-Trp,
 la séquence partielle
His-Leu-Asp-Ile-Ile-Trp
ou les séquences d'acides aminés cycliques
Cyclo-(DTrp-DAsp-Pro-DVal-Leu),
Cyclo-(DGlu-Ala-alloDIle-Leu-DTrp)

6. Procédé de préparation des composés suivant la revendication 1, caractérisé en ce qu'on fait réagir des composés de la formule générale (II)
X-CO-CR¹R²-A-CR³ R⁴-CO-X' (II)
dans laquelle R¹, R², R³, R⁴ et A ont la signification indiquée dans la revendication 1, et
X, X' représentent un groupe de départ,
avec des composés de la formule générale (III)
H-B (III)
et/ou de la formule générale (IV)
H-D (IV)
où B et D ont la signification indiquée dans la revendication 1,
et en ce qu'on conjugue éventuellement les composés ainsi préparés avec des substances qui s'enrichissent de manière sélective dans un tissu atteint de maladie ou dans des tumeurs, une liaison covalente étant nouée entre ces derniers, liaison qui s'effectue à la manière d'un amide dans le cas de substances contenant des groupes amino, comme des peptides, des protéines et des anticorps, ou à la manière d'un ester dans le cas de substances contenant des groupes hydroxy, comme des alcools, ou à la manière d'un imide dans le cas de substances contenant des groupes aldéhyde,
et en ce que les composés et produits de conjugaison ainsi préparés sont amenés à réagir avec du technétium-99m ou du Re sous la forme de pertechnétate ou de perrhénate, en présence d'un réducteur et éventuellement d'un ligand auxiliaire.

7. Composition radiopharmaceutique pour la représentation in vivo non invasive de récepteurs et d'un tissu contenant des récepteurs et/ou de plaques de type athérosclérose, caractérisée en ce qu'elle contient un composé ou un produit de conjugaison suivant l'une des revendications 1 à 5, ainsi qu'éventuellement les additifs courants en pharmacie galénique.
